# EUROPEAN PATENT SPECIFICATION

(11) **EP 2 134 857 B1**
(45) Date of publication and mention of the grant of the patent: **26.11.2014**
(21) Application number: 08742219.2
(22) Date of filing: 24.03.2008
(51) Int. Cl.: C12Q 1/04

(54) **DETECTION AND IDENTIFICATION OF MICROORGANISMS ON TRANSPARENT PERMEABLE MEMBRANES**
NACHWEIS UND IDENTIFIZIERUNG VON MIKROORGANISMEN AUF TRANSPARENTEN DURCHLÄSSIGEN MEMBRANEN
DÉTECTION ET IDENTIFICATION DE MICROORGANISMES SUR DES MEMBRANES PERMÉABLES TRANSPARENTES

(30) Priority: 22.03.2007 US 896321 P
(43) Date of publication of application: 23.12.2009
(73) Proprietor: Nanologix, Inc., Sharon PA 15146 (US)
(72) Inventor: GAZENKO, Sergey, V., Cincinnati, OH 45219 (US)
(74) Representative: Gowshall, Jonathan Vallance
(86) International application number: PCT/US2008/003826
(87) International publication number: WO 2008/118400

(56) References cited:
- US-A- 3 911 140
- US-A- 4 237 223
- US-A- 5 449 617
- US-A- 5 861 270
- US-A1- 2004 146 965
- US-A1- 2005 221 403
- MATTMAN L H ET AL: "Cellophane membranes in growth of L variants of the genus Proteus." APPLIED MICROBIOLOGY MAR 1958, vol. 6, no. 2, March 1958 (1958-03), pages 153-154, XP002576275 ISSN: 0003-6919
- TSE K-M ET AL: "MEMBRANE FILTER STAINING METHOD BACTERIAL PLATE COUNTS IN 24 HOURS" APPLIED AND ENVIRONMENTAL MICROBIOLOGY, vol. 48, no. 2, 1984, pages 433-434, XP002576276 ISSN: 0099-2240
- VAN POUCKE S O ET AL: "Rapid detection of fluorescent and chemiluminescent total coliforms and Escherichia coli on membrane filters", JOURNAL OF MICROBIOLOGICAL METHODS, ELSEVIER, AMSTERDAM, NL, vol. 42, no. 3, 1 November 2000 (2000-11-01), pages 233-244, XP002629220, ISSN: 0167-7012, DOI: 10.1016/S0167-7012(00)00193-7 [retrieved on 2000-10-19]
- VAN POUCKE, S.O. ET AL.: "Effects of the composition of bacteriological growth media on a chemiluminometric assay of beta-galactosidase in Escherichiacoli.", JOURNAL OF BIOLUMINESCENCE AND CHEMILUMINESCENCE, vol. 12, no. 3, May 1997 (1997-05), pages 165-175, ISSN: 0884-3996

## Description

### BACKGROUND OF THE INVENTION

### 1. Field of the Invention

The present disclosure relates generally to the field of biology and in particular to the field of rapidly growing, detecting, identifying, and enumerating microcolonies of microorganisms.

### 2. Description of the Prior Art

Modem microbiological analysis is based on two main trends: 1) analysis without preliminary growth and 2) analysis after such preliminary growth. The first trend includes a group of some immunological methods [e.g., immunofluorescence, radioimmunoassay, Enzyme ImmunoAssay (EIA) for single cell]; a group of methods based on DNA/RNA analysis via Polymerase Chain Reaction (PCR); and a group of Flow Cytometry methods [detection of single cell after labeling by fluorescent antibodies or fluorogenic substrates]. Artificial substrates are also used for detection and analysis of cells by microscopic means. Nevertheless, some of these methods (PCR and immunology) are not available for the detection of live cells. Flow Cytometry in combination with artificial (mainly fluorogenic) substrates is able to detect live cells but is very expensive, and requires highly skilled specialists in laboratory as well as concentrated samples. Yet a quick, inexpensive and effective method for detection and identification of live cells in different medical, biotechnological, food, agricultural, pharmaceutical, environmental, and military samples is still important for many human needs. And the frequently used tests that do use cells in microbiology [chromatography of fatty acids, Enzyme- Linked ImmunoSorbent Assay (ELISA), mass-spectrometry, Fourier transform infrared (FTIR) spectroscopy, and immuno-analyses] all require preliminary growth of a colony of microbes, which represents at least one time-consuming initial cell culture before detection and identification can take place.

Despite these high-tech alternatives, regular growth on a Petri plate is still the most common method used to detect microorganisms present in a sample. A typical analysis will perform several 10-fold dilutions of a sample followed by the application of one milliliter of the diluted sample distributed evenly over the surface of a nutrient agar. The quantity of 10-fold dilutions can usually be in any range, e.g., 1 to 12, with each dilution in a particular range being plated in a Petri plate in order to find a dilution suitable for counting microbial colonies. However, this might require possibly several to a dozen or more Petri plates. In practice, the correct dilution is found when the number of bacterial colonies on one count- able plate is not exceeds 250, as recommended by the FDA. In order to achieve this number, inoculated plates are incubated approximately 24-48 hours for bacteria and 72-120 hours for fungi. Thus a relatively long time is needed to form colonies easily visible to the naked eye. However, if the sample belongs to a time-sensitive biohazard incident, or a hospital patient in critical care, then time is of the essence and time- consuming incubation and serial testing can be a substantial burden with potentially life-threatening consequences.

Colonies appearing on solid nutrient media are simply counted for detection and enumeration of total microbial growth or are removed and analyzed according to traditional microbiological procedures, mass-spectrometry, FTIR spec- troscopy, chromatography, immunoassays, or PCR.

The removal of a suspicious colony and subsequent analysis of that colony by long and cumbersome traditional methods or complicated and expensive high-tech methods and instruments led to the invention of CHROMagar™ nutrient media containing special substances, substrates, and antibiotics that allow growth and simultaneous specific coloration of colonies of interest. The CHROMagar™ Candida, CHROMagar™ 0157, CHROMagar™ Salmonella, CHRO- Magar™ Staph. aureus and some other are currently used for identification of colonies of interest by pink, green, or blue color. The substances initiating coloration are collected in the cellular bodies, which causes growth problems. Therefore, colonies are atypically small and very often need prolonged incubation. Only regular-sized colonies can be detected because the color is weak, and small light absorption is use- less for microscopy. This means microcolonies (early stage of colony formation) cannot be detected with the use of CHRO- Magar™. Only species of interest and some number of other species can grow on CHROMagar™. Therefore CHROMagar™ is useless for the comprehensive (Total Viable Organ- isms) microbial detection and enumeration that is most commonly used in microbiology.

Overall, growth on Petri plates is a typical micro- biological operation used in medical, industrial, biotechnological, research, and pharmaceutical laboratories. World- wide, hundreds of millions of analyses are performed by these methods each year. Thus, there is an enormous demand for a more efficient, cost-effective, accurate, and timely product and procedure to allow detection, identification and enumeration for microbes.

US2004/14695 A relates to a Petri dish. US4237223 relates to a sheet for picking off microorganisms. Matman et al, Applied Microbiology, vol 6, no 2, 1958, p153-4, relates to cellophane membranes in microorganism separation. Tse et al, Applied and Environmental Microbiology, vol 48, no2, 1984, p433-4 relates to a membrane filter staining method. S.O Van Poucke et al, Journal of microbiological Method, vol 42, 2000, p. 233-244 relates to rapid detection of fluorescent and chemiluminescent total coliforms and Escherichia coli on membrane filters.

### BRIEF SUMMARY OF THE INVENTION

The invention is defined in the claims. The present disclosure provides one or more embodiments of an apparatus, a method, and a material that overcome the limitations of the prior art. In particular, the present disclosure accomplishes this with a device for detecting, identifying, or enumerating microorganisms that includes a container of media for providing nutrients to maintain growth of microorganisms and a porous element, or porous membrane, in contact with the nutrient media. The porous element itself allows nutrient media to pass through it to maintain growth for both traditional regular-sized colonies and microcolonies (together named "colonies"). And the porous element is transferable from the container to other locations for subsequent processing via indicators and further visual inspection.

In the method of the present invention, because the colonies are transferable via the porous element, the growth and indicator stages for the cells and microcolonies can be segregated for optimal results: the growth stage for fast cell growth without harmful indicators therein that retard growth, and the indicator stage for dedicated coloring and identification that effectively allow smaller size microcolonies to be detected quickly. Furthermore, if cells are evaluated at an early stage at primarily microcolony sizes, because they can be effectively detected with the present invention, then serial dilution is much less necessary. Thus the present invention overcomes the limitation of the prior art that essentially requires traditional regular size colonies from prolonged incubation, e.g., via CHROMa- gar, after serial dilution to prevent colonies from overgrowing each other. With fewer or no dilutions required for the present invention, results arrive faster and lab resources are con- served. In particular, the porous element may be moved, together with the colonies either on the porous element or on the nutrient media located superior to the porous element, to subsequent processing steps for a wide range of treatments including biochemical indicator processing or manual or automated visual detection, enumeration, and shape analysis (differentiation by shape) of the colored microcolonies, which are easily visible under a standard light microscope. To allow the sample to be inoculated on the media or on the porous element, the porous element has a property that allows the liquid portion of the sample to be communicated through it downward in the container. Then, to promote growth of cells trapped from the sample, the porous element has a property that allows the nutrients from the media to be communicated through it to the cells. Next, to allow the cells to be processed with indicator, the porous element has a property that allows a biochemical indicator such as different dyes or antibody conjugates to be communicated through it to the cells, while maintaining cell and microcolony integrity on the porous membrane without dissolving or washing away the microcolonies. Finally, to allow the cells and microcolonies to be inspected, the porous element has a visual property of transparency.

Two different embodiments of porous element positioning on the media exist. In a first embodiment, the porous element is disposed on top of the media layer and is not covered with any subsequent media. To effectuate transportation of cells, the porous membrane in this embodiment does not have porosity larger than the smallest cell desired to be evaluated in order to trap the cells above the porous element. Thus, when the porous element is removed from the media to be transported to subsequent processing, the cells and colonies accompany the porous element intact. This first embodiment is useful for fluorescent analysis because the porous element itself doesn't have background fluorescence. In a second embodiment, the porous element is disposed on top of the media layer and is subsequently covered with additional media. In this embodiment, the cells and microcolonies will reside on top of the additional media, and thus the porous element can have pore sizes larger than the smallest cell because the cells and microcolonies are trapped on the top surface of the additional media, superior to the porous element itself. Consequently, when the porous element is removed from the container, it takes the additional media above the porous element, along with microcolonies residing on that additional media. The second embodiment is useful for light absorbent coloration of microcolonies but not fluorescent detection because nutrient media itself has a high amount of background fluorescence.

To provide convenient testing, a test kit system of the invention includes a container of media for providing nutrients to pro- mote growth of microorganisms; a permeable membrane; and a secondary media chosen from the group consisting of: agarose, carrageenan, gelatin or other gels able to be the carrier with the ability to release indicator molecules, such as chromogenic substrates, fluorogenic substrates, or fluorescent or radio-labeled antibodies.

Overall, the present invention provides a much faster, more effective, less expensive, and more robust apparatus and means for: detection of total number of viable microcolonies or microorganisms (TYO) by intensely colored microcolonies; differentiation of one microcolony type from another by the easily visible shape of microcolonies, which are semi-specific for species or group of species; detecting and identifying microcolonies of antibiotic resistant microorganisms; identification by use of immunofluorescent or radio-immunolabeled antibodies; and detection, differentiation, and\or identification by light-absorbent means or by fluorescent means. These and other objects and advantages of the present invention will become apparent to those of ordinary skill in the art after having read the following detailed description of the preferred embodiments, which are also illustrated in the various drawing figures.

### BRIEF DESCRIPTION OF THE SEVERAL VIEWS OF THE DRAWINGS

The drawings included herewith are incorporated in and form a part of this specification. The drawings illustrate embodiments of the invention and, together with the description, serve to explain the principles of the invention. It should be understood that the drawings referred to in this description are not drawn to scale unless specifically noted as such.
FIG. 1 is a functional block diagram illustrating the spatial and functional relationship for two embodiments of the porosity and structural element and growth media element for evaluating a sample with cells in solution, in accordance with one embodiment of the present disclosure.
FIG. 2A is a container of agar media with multiple filtration elements thereon, in accordance with one embodiment of the present disclosure.
FIG. 2B is a cross-sectional view of multiple embodiments of the media and filtration element showing spatial relationships, in accordance with one embodiment of the present disclosure.
FIG. 3 is an illustration of the components and steps for a cell detection kit for detecting microcolonies by using, media and a transportable porous element, in accordance with one embodiment of the present disclosure.
FIG. 4 is an annotated picture of a culture in a container of agar media having multiple porous elements thereon along with a separate secondary media for coloration, in accordance with one embodiment of the present disclosure.
FIGS. 5A through 5H are photomicrographs of several different species of the genus Bacillus identified using the transferable porous element and the separate secondary media, in accordance with one embodiment of the present disclosure.
FIG. 6 is a photomicrograph of a species from the genus Listeria identified using the transferable porous element and the separate secondary media, in accordance with one embodiment of the present disclosure.
FIG. 7 is a photomicrograph of a species from the genus Staphylococcus identified using the transferable porous element and the separate secondary media, in accordance with one embodiment of the present disclosure.
FIG. 8 is a photomicrograph of a species from the genus Escherichia identified using the transferable porous element and the separate secondary media, in accordance with one embodiment of the present disclosure.
FIG. 9 is a photomicrograph of a species from the genus Pseudomonas identified using the transferable porous element and the separate secondary media, in accordance with one embodiment of the present disclosure.
FIG. 10 is a flowchart of the process for detecting and identifying cells and microcolonies, in accordance with one embodiment of the present disclosure.

### DETAILED DESCRIPTION OF THE INVENTION

The invention is defined in the claims. Reference will now be made in detail to the preferred embodiments of the invention. Examples of the preferred embodiment are illustrated in the accompanying drawings. In the following detailed description of the present disclosure, numerous specific details are set forth in order to provide a thorough understanding of the present disclosure. However, it will be apparent to one of ordinary skill in the art that the present disclosure may be practiced without these specific details. In other instances, well-known methods, procedures, components, and microbiological details have not been described in detail so as not to unnecessarily obscure aspects of the present disclosure.

### Terms And Explanations

Growth media. Growth media is a nutrient media used for growth of microcolonies within the context of the current invention. It can be general media for bacterial growth, such as Tryptic Soy Agar, media for fungi and yeast growth, such as Saboraud Dextrose Agar, or media used for growth of special groups of microorganisms, such as Cetrimide Agar used for growth of Pseudomonas species, or Mac- Conkey Agar used for the revealing of Gram-negative enteric pathogens. Growth media serves as a solid substrate for a porous element mounted on its surface.

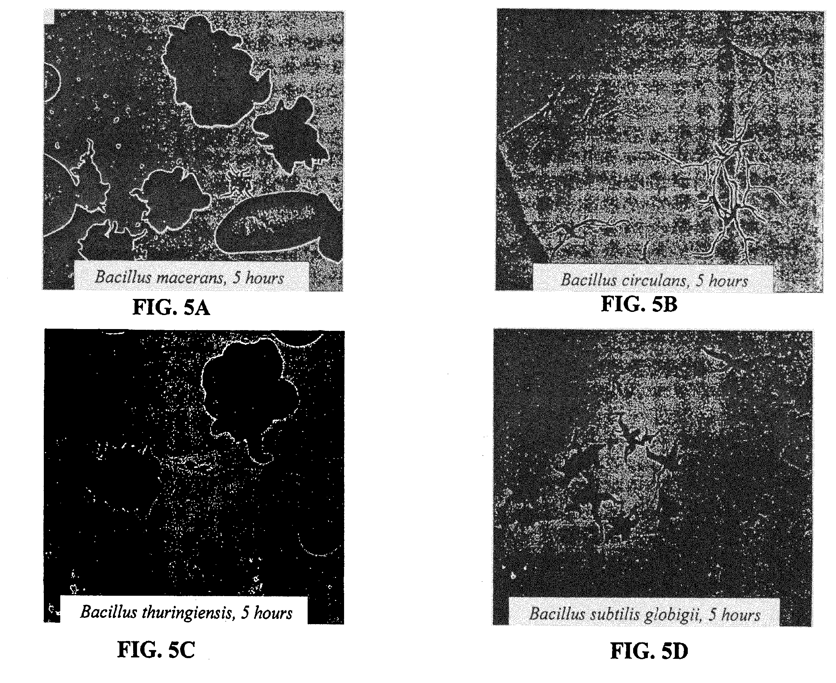

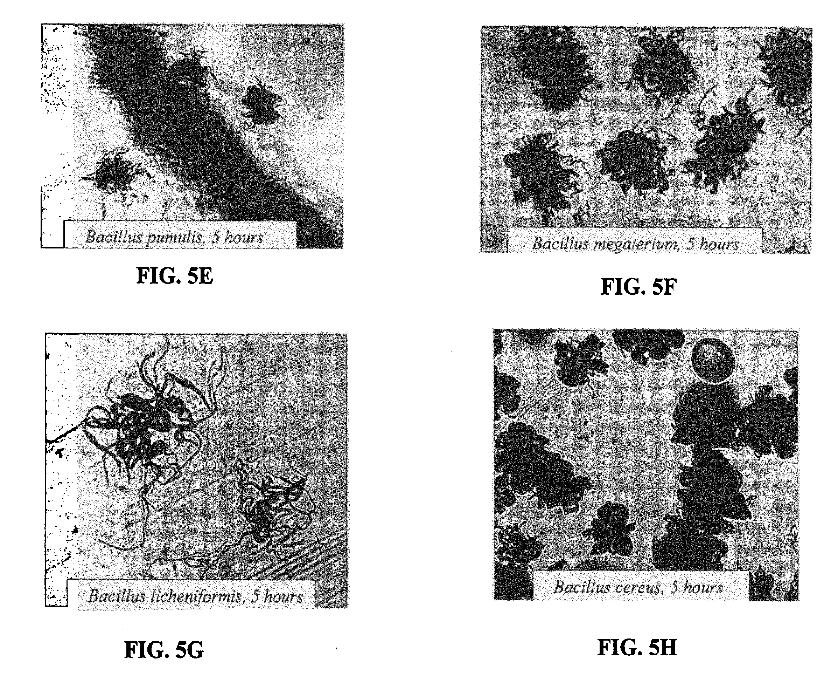

Microcolony. A microcolony is a small colony appearing after 3-6 hours of incubation. The typical size of microcolonies is 10-100 µm. They are colorless and invisible in a regular light microscope. Even colored microcolonies like yellow Staphylococcus aureus are not usually visible in a light microscope. Therefore, microcolonies typically need to be colored in order to become visible. The majority of microbial species produce microcolonies of specific shape during early stages of growth (3-6 hours). Quite often, differences in their shape are so evident that analysts have a good opportunity to differentiate species simply by the shape of a given microcolony (see FIGS. 5A-5H, and 6-9). Microcolonies consist of chains of cells which are not mechanically connected to each other. Therefore, all growth media and secondary media must not contain free liquids, lest the cells of the microcolonies dissociate from each other. All nutrient liquids and dissolved substances are bound by agarose or another polymeric gel.

Membrane. The membrane, or porous element, is an important material that allows growth and labeling of micro- colonies in order to make them visible and investigate their specific shapes. Requirements for this membrane are the following: must be light pellucid in order to allow light transmittance when used with a microscope; must be permeable to water and nutrient substances of nutrient media and indicator substances from secondary media in order to feed microorganisms during formation of microcolonies and subsequently color those microcolonies; must allow labeling molecules to penetrate and react with cells of microcolonies; must be non- permeable to cells (with a notable exception of porous membrane with a media layer superior thereto) so that cells will grow only on one side of membrane; must be resistant to external cellular enzymes in order to keep membrane structure intact and keep semi-permeability; must be autoclavable because membranes must be sterile before use; must be hydrophilic in order to allow water and the majority of sub- stances freely penetrate through it-hydrophobic membranes are strongly resistant to this kind of penetration; must be non-fluorescent and colorless because background fluorescence is strictly intolerable for fluorescent methods of analysis and color can mask color of microcolonies; must not contain any substances preventing or inhibiting microbial growth. In addition, the membrane must not contain visible particles or fibers like filtration membranes have because particles and fibers prevent free growth of microcolonies and are able to change time of growth and shape of microcolonies (with a notable exception of media layer superior to porous membrane). Experiments show that the best polymer for these purposes is regenerated cellulose and its derivatives like cellophane, cuprophane, and dialysis membranes. Other polymers showing the same qualities can be used. Many other types of materials for a membrane may be used such as organic or inorganic materials with naturally occurring pores, or with artificially created pores. Membrane may be transparent to transmit light for best viewing results, but may also be translucent, or white or other colors though they have less desirable results for viewing.

Secondary media. Secondary media is a gel, polymer, or BioNanoChannel™ plate serving as a carrier to indicator substances or labeled antibodies. Its main function is to carry indicator substances dissolved in liquid (water or buffer solutions) or antibodies and transfer them through the membrane to the microcolonies by simple diffusion. Gels like pure Agar (Agarose), Gelatin, Carrageenan, or other suitable polysaccharides with long polymeric molecules can be used to bind liquids and substances in semi-solid form. The use of just liquid solutions as indicators is usually avoided (with the notable exception of BioNanoChannel) because real liquid solutions penetrate the membrane rapidly and form large areas where all microcolonies dissociate, lose their shape, and then merge together again, forming one large clump of cells. Labeled antibodies, however, are relatively large molecules (IgG has size of approximately 150,000 Da) and cannot be released from a gel's polymeric structure easily. In this case, BioNanoChannel plates can be used instead of the noted gels. Each plate consists of millions of small nanochannels, each with a diameter of 10 µm or less. Thus, labeled antibodies are thousands of times smaller than nanochannels and can freely move in liquid into the nanochannels from one side of the plate. Once the liquid is in the nanochannels, however, it is trapped by strong capillary forces and does not move rapidly through the membrane and disintegrate microcolonies as free liquid does.

Indicators. In the realm of the current invention, indicators are substances that are able to specifically or non- specifically label microcolonies. They are chromogenic and/or fluorogenic substrates or their mixtures and labeled antibodies. Chromogenic and fluorogenic substrates produce light absorbent or fluorescent substances after a reaction with specific or non-specific enzyme, or a group of enzymes. Small molecules of these substrates can freely leave a gel, pass through the membrane, and react with enzymes of live cells. Antibodies labeled by fluorochromes or radioisotopes and introduced into a BioNanoChannel plate can freely leave nanochannels and pass through the membrane, e.g., as long as the membrane is 10⁶ Da pores or more. Non-reacted labeled antibodies are removed by transferring the membrane to another BioNanoChannel plate one or more times.

Detection, differentiation, and identification. The term "detection" is generally understood to mean the ability to reveal and count/enumerate any microcolonies independently of their taxonomic classifications. "Differentiation" means the ability of a used method to differentiate two or more species from each other on the membrane by their shape, color, or wavelength/color of fluorescence. "Identification" means the determination of genus and species of a given microcolony. Detection needs only chromogenic or fluorogenic substrates. Differentiation is based on the differences in shapes or colors of microcolonies. Identification requires the use of antibodies, preferably monoclonal.

### Detailed description of Figures

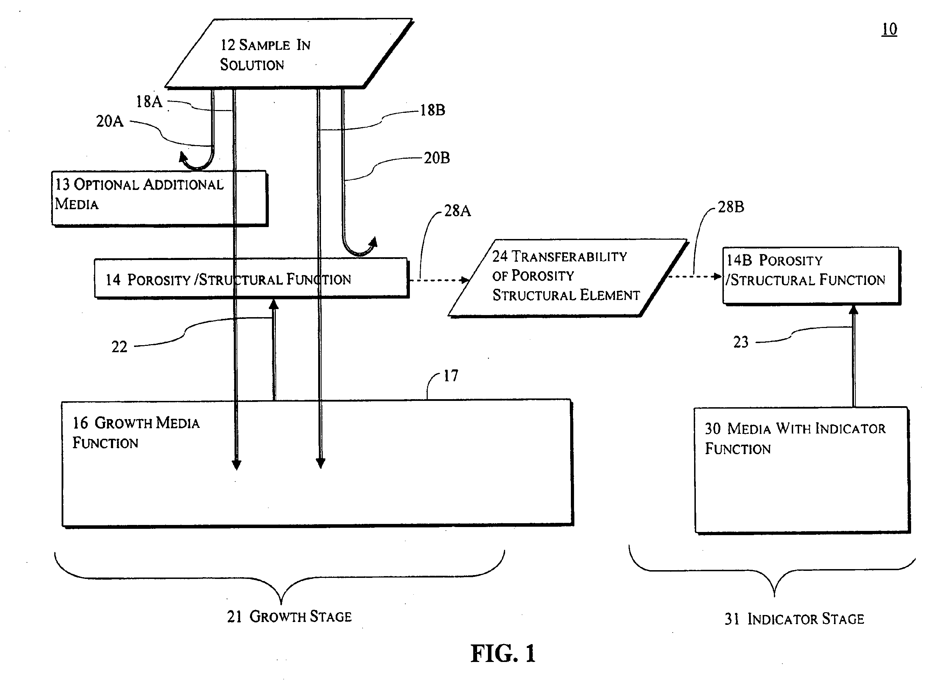

Referring now to FIG. 1, a functional block diagram 10 illustrating the spatial and functional relationship for two embodiments of the porosity and structural element and growth media element for evaluating a sample with cells in solution is shown, in accordance with one embodiment of the present disclosure. The first embodiment utilizes a porosity and structural element, or means, 14A disposed above top surface 17 of growth media means 16, with liquid portion 18B of sample solution 12 passing through filtration element means 14A and down through growth media 16 towards bottom of container holding growth media 16, while cell portion 20B of sample solution 12 is trapped or blocked from passing via porosity element means 14A by virtue of the pore size of membrane being smaller than size of desired cells to be blocked. Nutrients 22 from growth media means 16 is passed through membrane means 14 to cells 20B located on top of porosity element function 14A.

In contrast to the first embodiment, the second embodiment of FIG. 1 utilizes an additional optional layer of growth media 13 superior to, or disposed on, porosity and structural element means 14A. In this manner, liquid portion 18A of sample solution 12 passes through both additional layer growth media 13 and porosity element means 14A through growth media 16, but cell portion 20A of sample solution 12 is trapped or blocked from passing through additional layer of growth media 13 by virtue that, while liquid may permeate media, the cells cannot. Because of this, second embodiment membrane means 14A need not meet the requirement that porosity be smaller than cell size desired to be blocked. Rather, porosity element means 14A for this second embodiment has a primary function of providing structural support of additional layer growth media 13 and cells and microcolonies thereon for transfer means 24. A secondary function of porosity element means 14A in the second embodiment is for communicating nutrients 22 from growth media 16 to cells and microcolonies located on additional layer of growth media 13 which itself may have nutrients for the same purpose. Because of this, filtration element can be a much broader choice of materials and pore size to meet this structural requirement and communication of nutrients.

An important function of membrane means 14A for all embodiments of the invention is the transfer, or extraction, function 24 the filtered, trapped, or strained cells located on either porosity element means 14A or on additional layer of growth media 13 disposed thereon, for subsequent processing of trapped or blocked cells or microcolonies in indicator stage 31 on altered porosity/structural element 14B by the interaction with indicator 23. The ability to transfer viable cells and microcolonies while maintaining their integrity enables the growth and indicator stages for the cells and microcolonies to be segregated for optimal results: the growth stage 21 for fast cell growth without harmful indicators therein that would otherwise retard growth, and the indicator stage 31 for dedicated coloring and identification that effectively allow smaller size microcolonies to be detected quickly. Furthermore, if cells are evaluated at an early stage at primarily microcolony sizes, because they can be effectively detected with the present invention, then serial dilution is much less necessary. Thus the present invention overcomes the limitation of the prior art that essentially requires traditional regular size colonies from pro- longed incubation, e.g., via CHROMagar, after serial dilution to prevent colonies from overgrowing each other.

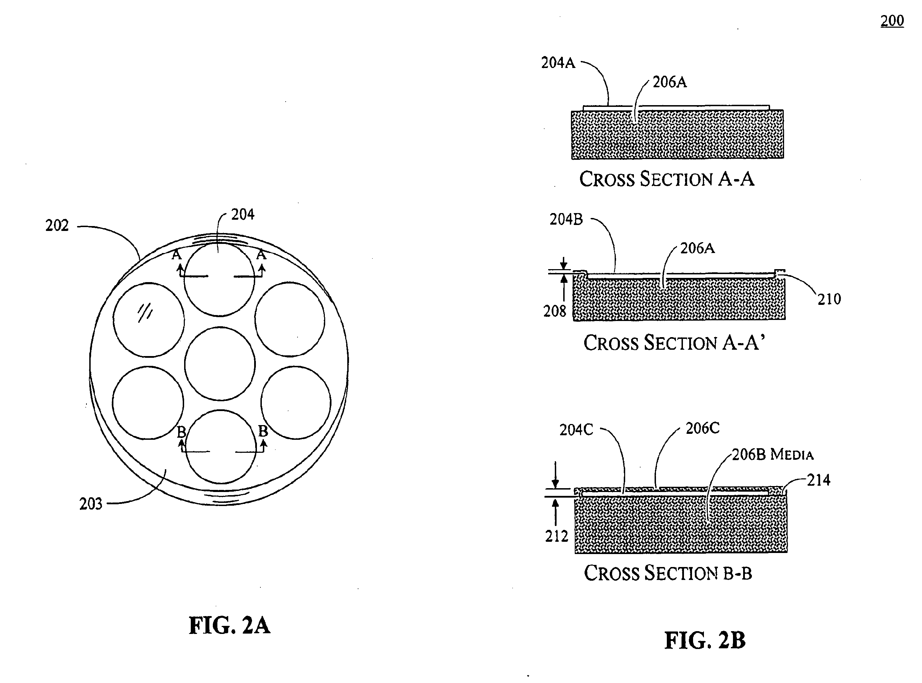

Referring now to FIG. 2A, a container 202 of agar media 203 with multiple membranes 204 thereon is shown, in accordance with one embodiment of the present disclosure, for parallel testing of a sample in a single container. Container 202 can be a conventional Petri plate provided to house nutrient media 203 that contains only substances that would not prevent or inhibit microbial growth in order to promote uninhibited growth of microcolonies for fastest response time on testing. Different embodiments of media 203 include a solid general purpose nutrient media, or a selective, differential, liquid or semi-solid media, or media with substances that only moderately affect microbial growth.

Porous Element 204 can be a porous element that allows sample solution and nutrient media to pass through it. Porous element can be a membrane, a permeable membrane, a dialysis membrane, or any type of existing filtration elements including cellulose, plastic material with holes, etc. The specific type of porous element 204 chosen depends upon whether a layer of additional media is placed upon it. Porous element can be pellucid, water-permeable, and permeable to nutrient substances and indicator substances, but optionally not permeable to microorganisms. In another embodiment, the porous element is a permeable membrane that has one or more of the following characteristics: autoclavable, hydrophilic, non-fluorescent, colorless, resistance to secreted cellular enzymes, and has regular or non-regular pores within the range of: 1000-10⁷ Daltons (Da). The porous element material may be selected from the group consisting of: regenerated cellulose, cellophane, cuprophane, dialysis membrane, and other material that have appropriate size pores and that meet other properties mentioned herein. It is known that dialysis membranes are highly permeable for substances/molecules of a certain size, such as the nutrients needed to grow micro- organisms. Pores of a dialysis membrane can range from 1,000 to several hundred thousand Da (Daltons). The large size of pores allows many substances to transfer through the membrane. However, polymers with long molecules, such as the galactose polymer in galactose agar used in microbiological solid media cannot penetrate dialysis membranes. Experiments show that microorganisms can grow on the surface of a dialysis membrane when the membrane is placed on top of solid nutrient media because of easy penetration of nutrients through the membrane to growing cells. Dialysis membranes on a surface of solid nutrient media or installed in a layer of agar allow transfer of colonies or micro-colonies appearing on its surface to another surface filled with indicator sub- stances.

In another embodiment porous element can be a wide variety of regular filter elements provided primarily for structural and fluid communication functions and not for a cell trapping or blocking function, which function is instead performed by additional media layer on top of filter element as provide solely by first embodiment of FIG. 1.

Referring now to FIG. 2B, a cross-sectional view 200 of multiple embodiments of the media and porous element showing spatial relationship is shown, in accordance with one embodiment of the present disclosure. Cross section A-A illustrates a porous element 204A simply disposed on top of solid nutrient media 206A, allowing for easy transfer- ability from growth stage on solid nutrient media 206A but possibly detracting from an even application of cells from sample onto top surface of porous element. In contrast, alter- native embodiment cross-section A-A' provides a cavity 210 in top layer of solid nutrient media with a depth sufficient to allow for recession of porous element 204B from zero to some nominal value 208, e.g., 0.1-5 mm, below the surface of solid nutrient media, thereby providing a shallow well in which sample can be pipetted and spread with assurance of a controlled surface area. Cavity 210 can be formed by a hot element in the shape of the cavity, by molding the liquid nutrient media around a temporary spacer in the shape of the cavity which is removed upon solidification of the media. Cross section B-B provides an embodiment where a first portion of solid nutrient media is deposited then solidified, followed by placement of porous element 204C, followed by a second layer of nutrient media 206C which covers porous element in a layer depth of 212, leaving a non-visible seam interface 214 between the first and second layer of nutrient media 206B and C. The present invention is well-suited to a wide variety of methods and procedures for placing porous element on or within nutrient media.

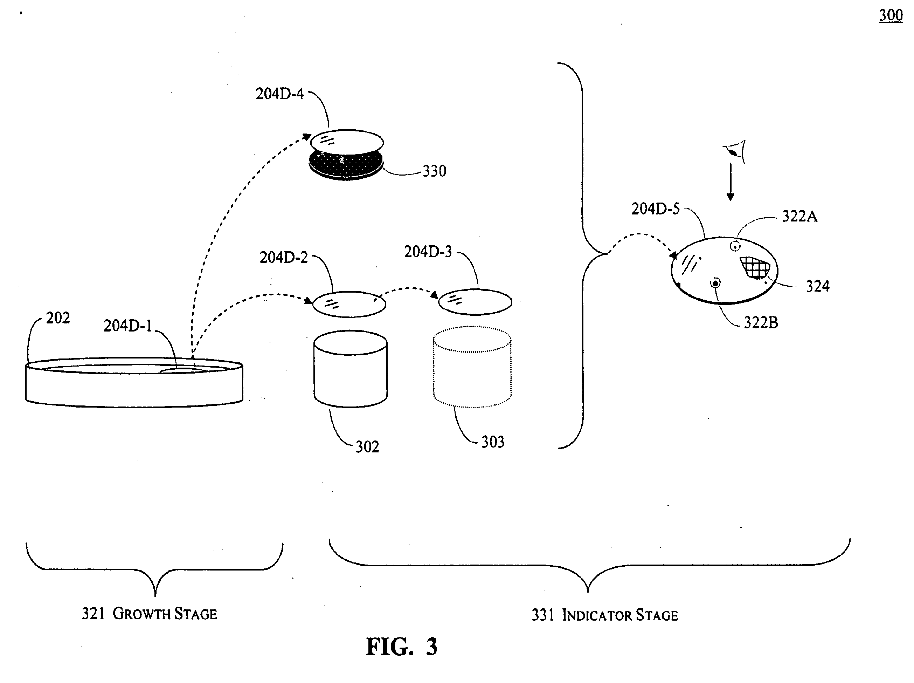

Referring now to FIG. 3, an illustration of the components and steps for a cell detection kit 300 for detecting microcolonies by using media and a transportable porous element is shown, in accordance with one embodiment of the present disclosure. Porous element 204D-1, after being removed from container 202 containing growth media in growth stage 321, is transferred to secondary media 302 with indicator substance in indicator stage 331 which potentially alters cells and microcolonies on porous element 204D-2. In one embodiment, two or more indicator steps are utilized to satisfy a protocol for indicating a desired cell or microcolony, e.g., second indicator step using media 303 which further transfers indicator substance to potentially modify cells or microcolonies on porous element 204D-3. Secondary media is chosen from the group consisting of agarose, carrageenan, gelatin, and a gel able to be the carrier with the ability to release indicator molecules. In another embodiment, the test kit system provides a concentration of gel in a range of approximately 0.1%-10% in a solvent of water, buffer, sodium chloride solution, or liquid nutrient media. Indicator for microcolonies can be a chromogenic substrate, fluorogenic substrate, or fluorescent or radio-labeled antibodies. For example secondary media can be pure agar filled with methyltiazoltetrazolium bromide (MTT), which will color the micro-colonies dark violet. Non-colored micro-colonies/ cells are essentially invisible in the microscope. Many other substances can be used for detection and differentiation of microorganisms, including those that are toxic to cells and restrict growth. Many rapid and simple methods of detection and identification can be transformed to be used with this method.

After processing with indicator material and processes, a final porous element 204D-5 with appropriately colored microcolonies 322A and 322B may be visually observed and counted, e.g., under a light microscope or using an automated hardware and software image identification, recognition, and enumeration system with magnification. Optional grid 324, made by either printed or geometrically formed by scoring or etching, thereby providing a convenient counting reference or metric to ratio the surface area counted to arrive at a final microcolony population.

In an alternative assay, colonies and microcolonies may be identified with antibodies labeled with fluorescent dyes. This assay can be accomplished, in one embodiment, using a BioNanoChannel ("BNC") detection device 330 as the carrier for the antibody. In this case, antibodies are provided in the liquid solution inside the nanochannel, onto which is placed the porous element with the colonies. The top of the BNC provides support for the porous element 204D-4 to maintain integrity and cohesiveness of existing colonies, while discretely allowing the large molecules of antibodies to float in the nanochannel and be communicated to any colonies on the porous element 204D-4 directly overhead the top of the open channel. A porous membrane with sufficiently large pores, e.g., 1⁶ Da or larger or as appropriate for the specific antibody desired to be transferred to a colony. Only the appropriate colony (antigen) will react when the antibody comes in contact with it. Excess labeled antibody present on the porous element can be removed by transferring, one or more times, the porous element to another BNC having solvent or buffer without fluorescent molecules. In contrast, the large antibody molecules would not have motility in agar or gel because their large size would impede them. Furthermore, placing the porous element directly onto an open container of liquid media with antibodies would not maintain the integrity of the colonies, as the membrane would become saturated, swamped, or washed away by the fluid, thereby dissolving and dislocating the colonies. Thus, using the transferrable porous membrane of the present disclosure in conjunction with a BNC plate, that provides a support for the porous membrane and a delivery mechanism for the labeled antibody, provides a very effective solution that overcomes the prior art limitations.

The BNC is a plate that contains a multiplicity of cylindrical and parallel micro channels open from one or both ends, and having a micro-channel diameter of approximately 1-30 µm with a length of approximately 100-1000 µm and a number of micro-channels per centimeter2 of approximately 100,000-1,000,000, long (diameter/length=1/10-1/100). Further details and descriptions of the BioNanoChannel apparatus and methods are provided in co-owned U.S. patent application Ser. No. 11/109,857 to Sergey Gazenko, entitled "Device For Rapid Detection And Identification Of Single Microorganisms Without Preliminary Growth"

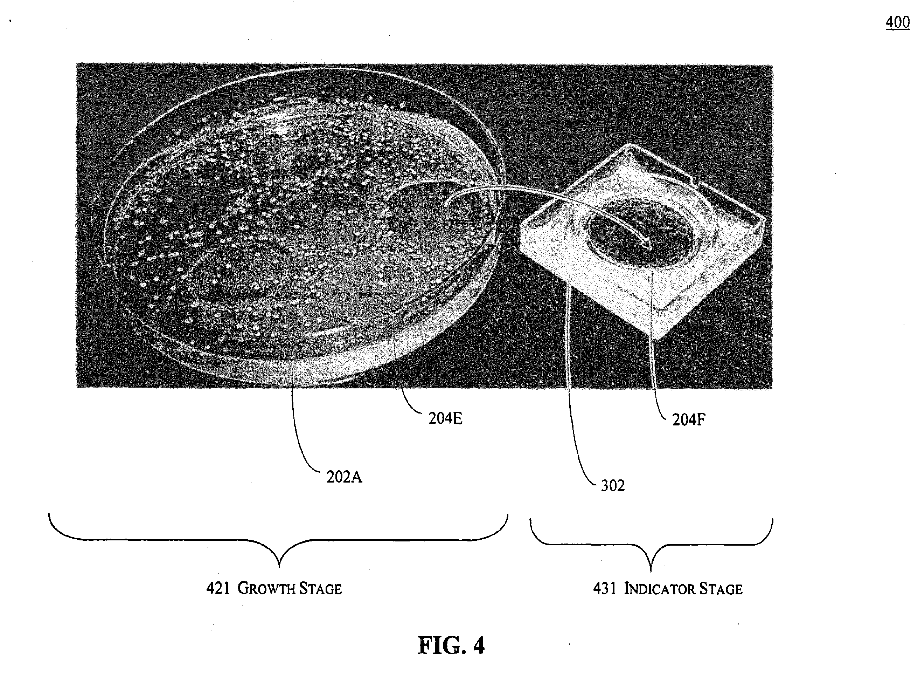

Referring now to FIG. 4, an annotated picture 400 of a culture in a container of agar media having multiple porous elements thereon along with a separate secondary media for coloration is shown, in accordance with one embodiment of the present disclosure. Petri plate container 202A of nutrient media with multiple porous element such as 204E and a culture thereon illustrates the success of the microcolony growth in the growth stage 421 as well as the clarity of the porous element and the ease of transfer of porous element 204F to container 302 in indicator stage 431 with secondary indication media therein, which indicator clearly effectuates the darkened microcolonies, otherwise difficult to detect in the indicator stage of nutrient-only media container 202A.

Referring now to FIGS. 5A through 5H photomicrographs of several different species of the genus Bacillus identified using the transferable porous element and separate secondary media are shown, in accordance with one embodiment of the present disclosure.

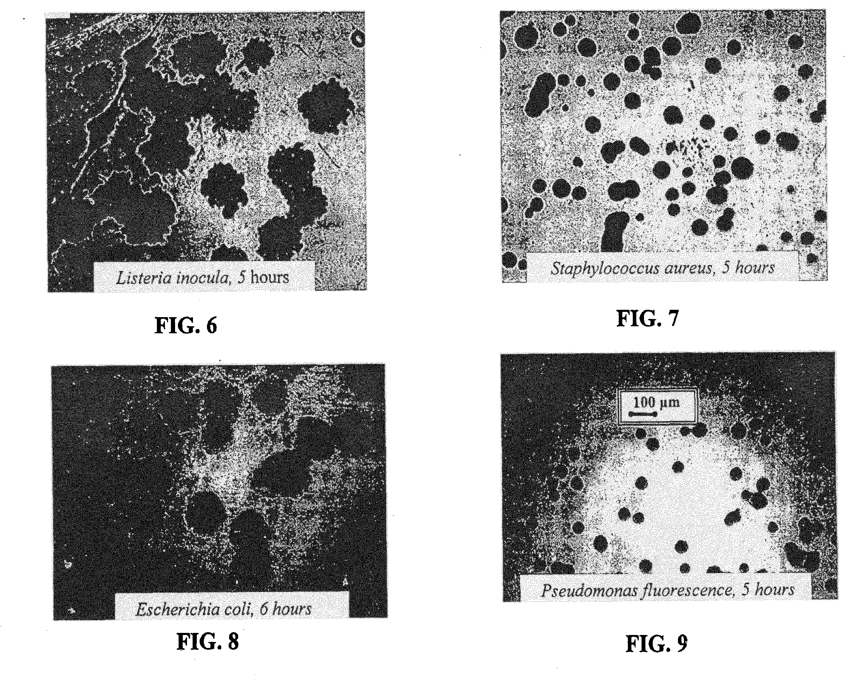

Referring now to FIG. 6, a photomicrograph of a species from the genus Listeria identified using the transfer- able porous element and separate secondary media is shown, in accordance with one embodiment of the present disclosure.

Referring now to FIG. 7, a photomicrograph of a species from the genus Staphylococcus identified using the transferable porous element and separate secondary media is shown, in accordance with one embodiment of the present disclosure.

Referring now to FIG. 8, a photomicrograph of a species from the genus Escherichia identified using the transferable porous element and separate secondary media is shown, in accordance with one embodiment of the present disclosure.

Referring now to FIG. 9, photomicrograph of a species from the genus Pseudomonas identified using the transferable porous element and separate secondary media is shown, in accordance with one embodiment of the present disclosure.

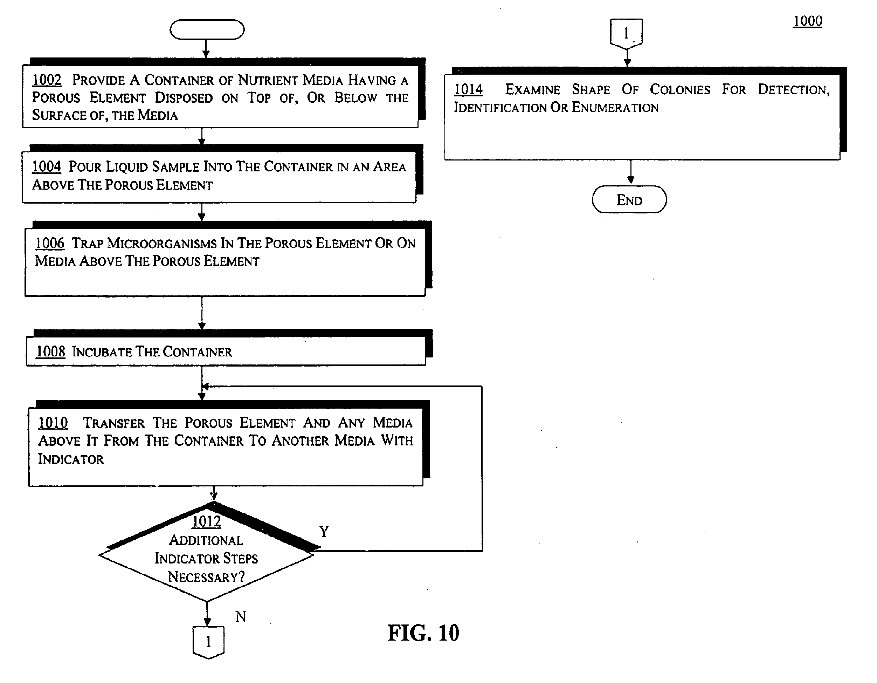

Referring now to FIG. 10, a flowchart 1000 of the process for detecting and identifying colonies, including microcolonies and regular colonies, is shown in accordance with one embodiment of the present invention. To begin, step 1002 provides a container of nutrient media having a porous element disposed on top of, or below, the surface of, the nutrient media, as shown in FIG. 2B. Subsequently, in step 1004 liquid sample is poured, or pipetted into the container in an area above the porous element. The sample may be treated as a spread plate sample to be spread across the entire Petri plate, or it may be concentrated only on the porous element, e.g., as shown in cross-section A-A' wherein porous element 204B is recessed into cavity 210, thereby restricting the sample to only being exposed to porous element 204B. Then in step 1006, the microorganisms are trapped or blocked in the porous element or alternatively, on an additional media layer on top of porous element, the latter shown in cross section B-B of FIG. 2B. Step 1008 provides for incubation to develop microcolonies, albeit much shorter than a typical prior art incubation for regular-sized colonies. In subsequent step 1010 the porous element and any media above it is transferred from the container with nutrient media to another media with indicator for the purpose of assaying for detection and/or identification of microorganisms. In step 1012 an inquiry determines whether additional indicator steps are necessary. If they are, as is often the case, step 1010 is repeated, but most likely with a new or complementary indicator agent. Else, step 1014 provides for a final step of examining shape or color of colonies for detecting, identifying, or enumerating appropriate microcolonies.

### EXAMPLES

Detection of total number of viable microorganisms (TYO) is one of the most needed laboratory procedures in the world. It shows the presence and level of microbial contamination in the food, biotechnological, environmental, and other industries. A regular TYO is conducted by doing several (3-12) 10-fold dilutions of a sample in buffer, liquid nutrient media, or 0.9% NaCl solution and then spreading one milliliter of each dilution on the surface of regular Petri plates filled with an appropriate nutrient agar. Plates are incubated 24-48 hours at 32-37° C. After this plates are removed from the incubator and colonies are enumerated. This procedure needs 3-12 tubes for dilutions, 3-12 Petri plates, and long term incubation. Results appear only after 24-48 hours. This simple procedure is repeated hundreds of millions of times worldwide each year. The method of the present disclosure, described herein is much faster (3-6 hours), does not require 10-fold dilutions, and needs only 1-2plates with pre-installed membranes. Installation of the membranes is done by placing several round-shaped, sterilized (121° C., 15 min.) dialysis membranes (pore size 12,000-18,000 Da) on the surface of nutrient agar solidified in a Petri plate. Other membranes with a different pore size or cellophane membranes can be utilized as well. The entire surface of the plate with membranes is then covered by hot (80-90° C.) solid nutrient media for 1-2 seconds; the excess media not solidified after 1-2 seconds is poured out. This means that all membranes will be covered by a thin (0.1-0.2 mm) layer of nutrient media. The upper layer has a free exchange with the lower media, meaning that all water and nutrient substances except the polymer Agarose can be transferred between the two layers.

The procedure according to the current invention is the following: one milliliter of sample is transferred to and spread on the surface of the Petri plate with installed membranes (FIG. 2). Liquid from the sample is absorbed in the media, the plate is incubated, and live cells, if any, begin growth and microcolony formation. Experiments show that all investigated bacteria form microcolonies within 5 hours at 35-37° C. These microcolonies consist of several tens to several hundreds of non-colored and essentially invisible cells. In order to color them, the membrane is peeled from the plate using sterile forceps and is transferred to a secondary media. The secondary media according to a standard method is 1% pure Agarose in 0.15M Phosphoric buffer, pH 7.5 with chromogenic substrate 3-(4,5-Dimethylthiazolyl-2)-3,5- diphenyltetrazolium bromide in concentration 0.7 mg per ml. This slightly yellow substrate has the ability to become dark violet after a reaction with dehydrogenases of live cells. This substance has the ability to change the color of all known aerobic and anaerobic bacteria and yeasts, because it reacts with the final step of the electron-transport chain of microorganisms. In other words, it does not block the beginning or middle part of the respiration chain, which can lead to cellular early death. Thus, a color product is collected in large concentrations on the surface of cellular mesosomes (location of dehydrogenases inside cells), imparting a dark violet or black color to the cellular bodies. The time needed to color micro- colonies is typically in a range of 10-20 min at 30-40° C. This time is enough to free the molecules of the substrate and allow them to penetrate the membrane and thin layer of solid nutrient media by diffusion and react with dehydrogenases. After the reaction is complete, the plate with secondary media and colored microcolonies is moved under a light microscope and microcolonies are enumerated. The size and shape of microcolonies can be an additional characteristic for species differentiation (FIG. 5.1-5.8).

This method needs only 5-6 hours and many less materials and manipulations than the prior art method. It is important to note that the present example does not need numerous 10-fold dilutions, but only 1-2 dilutions in the case of an extremely high concentration. This convenience is possible because of the relatively small size of microcolonies: the concentration of cells in a sample can be millions per milliliter, but all microcolonies will grow separately without overlapping because the square of the size of one microcolony is thousands of times smaller than the square of the size of a regular colony.

### Fluorescent Embodiment

Membranes used in the current method are made from regenerated cellulose, which is non-fluorescent and pellucid material. Nevertheless, nutrient media itself contains highly fluorescent substances. This restricts the use of a thin layer of nutrient media above the porous element, or membrane, as it was described in one embodiment. The recommended membrane in this case is a 100,000 Da dialysis membrane. The relatively large size of pores in this membrane allows satisfactory growth of microcolonies even without the thin layer, of media above them. Enhancement of humidity is also helpful in this embodiment because it creates more favor- able conditions (microclimate) for microcolony formation. After 5-6 hours of growth, the membrane is removed to a secondary media filled with one or more fluorogenic substrates. For TYO detection, a freshly prepared mixture of 4-methylumbelliferyl phosphate and 4-methylumbelliferyl acetate in a concentration of 0.1 mg per mL of Acetone is used. This mixture is poured on the surface of a secondary media (1% Agarose in distilled water) for several minutes before the membrane containing microcolonies is mounted above. Incubation takes 10-15 minutes at 35-40° C. All microcolonies obtain blue fluorescence that is very visible in a fluorescent microscope because the mixture of substrates used reacts with a large number of phosphatases and esterases present in all live cells. The light-pellucid, non-fluorescent membrane allows use of an inexpensive fluorescent micro- scope with exciting UV (e.g., wavelength 350-380 nm) light directed from bottom to top, though expensive epi-fluorescent microscopes can be used as well.

### Immunofluorescent Embodiment; Identification

Identification of microorganisms by antibodies labeled with fluorochromes (FITC, Fluorescein, Rodamine, or other) can be performed by use of the porous element, or membrane, without a layer of solid nutrient media above. The immunoglobulins used as antibodies are a very large protein molecular complex. For example, the frequently used immunoglobulin G (IgG) is around 150,000 Da. Such a large complex must be able to pass through the membrane and react (antigen-antibody interaction) with the expressed surface antigens of cells. Therefore, the size of membrane pores is larger, e.g., 10⁶ Da or larger.

To perform the method, a sample, containing live cells of different species, including target species, is spread on the surface of the membrane. Liquid soaks into the agar growth media and the plate is incubated 5-6 hours at 35-37° C. in highly humid conditions in order to form microcolonies. After this, the membrane with microcolonies on its surface is placed onto a BioNanoChannel plate filled with a solution containing labeled antibody complimentary to target micro- organisms (e.g. IgG-FITC). Molecules of labeled antibody float freely inside nanochannels, each nanochannel having a diameter of 10 µM and length of 500 µM. Around 2.5 million nanochannels cover the BioNanoChannel plate, which has a diameter of 25 mm. Thus, the solution with antibodies is "trapped" inside a massive collection of tiny glass channels. This is important to prevent wet areas with extra liquid which can dissolve microcolonies and mix target cells with cells of other species. Use of a BioNanoChannel plate is also important because it allows the large IgG molecules to roam freely, whereas the Agarose used in other embodiments (those for small molecules) would not be able to release IgG from its polymeric structure.

Molecules of labeled IgG reach all microcolonies by diffusion and react with target antigens. This process can take 0.5-1.0 hour at room temperature. Non-reacted molecules can be removed by placing the membrane on another Bio- NanoChannel™ plate filled with washing solution (PBS, pH 7.5) for 10-20 minutes. After this, the membrane with labeled and non-labeled (fluorescent and not or weakly fluorescent) microcolonies is placed under a fluorescent microscope and fluorescent (target) microcolonies are detected and enumerated.

Detection, identification, and/or microcolony shape recognition can be accomplished by the operator using a visual method or by the use of image identifiers. Different kinds of image identifiers currently being used are based on CCD cameras and computer image identification programs and are combined with a microscope and computer to create an image and analyze it.

Identification of microcolonies can be improved by the use of micromanipulators, which remove a microcolony chosen by shape or fluorescence, followed by the microcolony's identification by PCR or other suitable methods. Removal of microcolonies can be accomplished by different methods. However, identification of a microcolony by PCR does not necessarily mean the specific removal a given microcolony is necessary because PCR methods allow recognition of the DNA of a target organism on the background DNA of other species.

Membranes of different pore sizes and other qualities are useful for virology, especially in cases when cell cultures are used for growth and detection of viruses through holes in the cellular layers.

### Quality of Decontamination

Decontamination of indoor environments after an epidemic, the presence of person(s) with a deadly disease, or a terrorist or foreign military biological attack are all very important dilemmas for the average microbiologist. The presence and amount of surviving cells after decontamination defines the possibility for further use of decontaminated facilities. This analysis must be completed in a very short time, especially if a facility of important public use is involved (airports, government or military facilities, or other places of high public access and importance). Only live/surviving cells must be detected in this circumstance. And only the actual growth of cells is known to be the most reliable method of detecting live microorganisms. Regular growth takes 24-48 hours or more for creating a colony. The current invention allows the same results in 5-6 hours, is much less expensive, and requires much less manual processing; thereby meaning a larger number of samples can be analyzed. For example, if an area was contaminated by Bacillus anthracis spores and decontamination kills all vegetative cells and spores, then only spores of Bacillus can survive. Spores of Clostridium, Actinomycetes and Fungi are not grown on media used for B. anthracis because of different reasons. Thus, the only species of Bacillus that will be growing on this media is B. anthracis. TSA can be used in this case. Liquid samples from different parts of a facility are placed on TSA with the membrane with thin layer of TSA above. After 5 hours of incubation at 37° C. membranes with possible microcolonies are transferred to plates with 3-(4,5-Dimethylthiaz- olyl-2)-3,5-diphenyltetrazolium bromide (1% of pure Agarose in PBS pH 7.5). In 10-20 minutes intensely colored microcolonies of a specific shape can be recognized from other Bacillus species (for example, FIG. 5.4 shows what looks like Bacillus subtilis globigii, known as a model in Bae. anthracis research). These microcolonies can be removed and analyzed by rapid PCR (1-1.5 hours) for confirmation of B. anthracis surviving decontamination. Thus, permission to continue use of the facility can be granted after 6-8 hours instead of 24-48 or more hours.

### Detection of Antibiotic-Resistant Microorganisms

Detection of antibiotic resistant microorganisms in human samples and as nosocomial infections (Staphylococcus aureus, Pseudomonas aeruginosa, Klebsiella oxitoca and other) is a fast growing medical problem. The life of an infected person strongly depends on rapid diagnostics of an antibiotic resistant strain. Growth and creating of a colony in the presence of an antibiotic is only one reliable method broadly used in diagnostics of antibiotic resistant strains. It currently takes 24 hours to find growth/colonies on plates with nutrient media and antibiotic (CHROMagar, MRSA or other). Limiting this analysis to 6-8 hours can save tens of thousands of lives worldwide per year. As an example, the general method for the rapid detection of Staphylococcus aureus is following: A sample (blood or environmental), presumably containing antibiotic-resistant S. aureus, is spread on a plate filled by methicillin (MRSA media) with installed membranes. After 5-6 hours the membrane is transferred to a plate with Agarose and a chromogenic substrate. After 10-15 minutes all microcolonies obtain a dark violet color and become visible. Microcolonies of specific shape (FIG. 7) are recognized as antibiotic resistant S. aureus.

### Rapid Detection of E. coli

Presence of E. coli in food and environmental samples is known to be a reliable characteristic of fecal contamination. Only live cells are important for obtaining enumeration results; immunological and PCR methods are ineffective in this case. The ability to form a colony is the most reliable characteristic of live E. coli. For growth and detection of E. coli, MacConkey agar is broadly used for Gram-negative, Lactose-positive microorganisms. Alternatively, CIRCLEGROW® media is used for rapid growth of E. coli. LB agar can also be used. All of these media are suitable for growth of microcolonies of E. coli and some other microbes present in a sample. The porous element, or membrane, installed on the media surface must not have a thin layer of agar media on top and must have appropriate size pores, e.g., 50,000-100,000 Da. E. coli forms well-visible microcolonies after 4-6 hours of incubation at 37° C. (FIG. 8). After incubation period, the membrane containing both E. coli and microcolonies of other species is transferred to a BioNanoChannel plate filled by 4-Methylumbelliferyl- -D-galactopyranoside (0.1 mg/ml in 35% Ethanol). After 5-10 minutes of incubation, microcolonies of E. coli obtain bright blue fluorescence under a fluorescent microscope at exciting UV 350-380 nm. Other microcolonies remain colorless.

### Alternative Embodiments

The present description is applicable to a wide variety of applications and is not limited to any particular type of assay, media, filter, membrane or microorganism described in the present disclosure. In view of the embodiments described herein, the present disclosure has been shown to provide a method and apparatus that overcomes the limitations of the prior art, such as numerous serial dilutions, excessive time and lab resource consumption, and weak results.

For example, aspects of the present disclosure can be adapted to detect viruses growing on a thin layer of prokaryotic or eukaryotic cells and producing clear holes on the surface in the place where viruses propagated. In this case, coloration can be colored cells that are intact and not infected by virus and small clear spaces, or plaque, where cells are infected and disintegrated by virus with no viable cells to color.

## Claims

1. A method for growing, detecting, and identifying or enumerating microorganisms, the method comprising the steps of:
providing a container of nutrient media with a porous element disposed on top of, or under the top surface of, the media, wherein the media has nutrients to maintain growth of microorganisms, and wherein the porous element has pores ranging from 1,000 to 10⁷ Daltons;
pouring a liquid sample into the container in an area above the porous element;
trapping microorganisms in the porous element or on media above the porous element;
incubating the container for a time sufficient to grow a microcolony , said growth of the microcolony referred to as the growth stage;
transferring the porous element and any media above it from the container to a secondary media for purposes of evaluating, detecting and identifying the microorganisms, said evaluation, detection and identification of the microorganisms referred to as the indicator stage;; and
examining the microcolonies for growth, detection, identification or enumeration of microorganisms, wherein said method for growing, detecting, and identifying or enumerating microorganisms takes up to six hours, wherein the growth stage and the indicator stage are segregated for optimal results, and wherein the growth stage permits fast cell growth without harmful indicators therein that retard growth, and the indicator stage permits dedicated coloring and identification that effectively allows for smaller size microcolonies to be detected quickly.

2. The method recited in claim 1, wherein the porous element is a permeable membrane selected from the group consisting of polymers, such as regenerated cellulose, cellophane or cuprophane, and dialysis membranes.

3. The method recited in claim 1, wherein the examination of the microcolonies is for identification of microorganisms, using monoclonal antibodies.

4. The method recited in claim 1 wherein the examining step is performed manually or with an automated image detection and recognition system.

5. The method recited in claim 1 further comprising the step of detecting the total number of viable microcolonies (TVO).

6. The method recited in claim 1, wherein the secondary media contains an indicator substance to assist with the identification of microorganisms on the porous element.

7. The method recited in claim 1 wherein the porous element traps either non-filterable or filterable samples.

8. Use of a device in a method according to any one of claims 1 to 7, comprising:
a container of nutrient media for providing nutrients to maintain growth of microorganisms;
a porous element in contact with the nutrient media, wherein the porous element allows nutrient media to pass through it to maintain cell growth, wherein the porous element is transferable from the container for subsequent processing with indicators, and the porous element is pellucid, water-permeable, and permeable to nutrient substances.

9. The use of a device recited in claim 8, wherein the porous element is a permeable membrane that is impermeable to microorganisms but is autoclavable, hydrophilic, non-fluorescent, colorless, and is resistant to secreted cellular enzymes.

10. The use of a device recited in claim 8, wherein the porous element is disposed above the media in the container with or without a superior layer of additional media.

11. The use of a device recited in claim 8, wherein the porous element is a permeable membrane with regular or non-regular pores within the range of: 10⁶-10⁷ Da.

12. The use of a device recited in claim 8, wherein the media is in a solid, semi-solid, or liquid state and wherein the porous element communicates nutrients from the media to microorganisms located above or on the porous element.

13. The use of a device recited in claim 8, further comprising:
a plurality of porous elements disposed on or in the media in a parallel arrangement for parallel testing.

14. A test kit system for growing, detecting, and identifying or enumerating microorganisms, the test kit comprising:
a device defined in any one of claims 8 to 13;
a secondary media acting as a carrier to release indicator molecules to color colonies; and
a bionanochannel detection plate having a multiplicity of cylindrical and parallel microchannels open from one or both ends, each microchannel having a diameter of 1 µm to 30 µm and a length of 100 µm to 1000 µm.

15. A test kit system according to claim 4, wherein the secondary media is selected from the group consisting of agarose, carrageenan, gelatin and a gel able to communicate, and wherein the concentration of gel ranges from 0.1 % to 10% in a solvent of water, buffer, sodium chloride solution, or liquid nutrient media

## Patentansprüche

1. Verfahren zur Züchtung, Detektion und Identifizierung oder Zählung von Mikroorganismen, wobei das Verfahren die folgenden Schritte umfasst:
Bereitstellung eines Nährmediumbehälters mit einem porösen Element, das oben auf dem Medium oder unter der oberen Oberfläche des Mediums angeordnet ist, wobei das Medium Nährstoffe zur Aufrechterhaltung des Wachstums von Mikroorganismen aufweist und wobei das poröse Element von 1.000 bis 10⁷ Dalton große Poren aufweist;
Gießen einer flüssigen Probe in den Behälter in einem Bereich oberhalb des porösen Elements;
Einfangen von Mikroorganismen im porösen Element oder auf Medien oberhalb des porösen Elements;
Inkubieren des Behälters für eine Zeit, die zur Züchtung einer Mikrokolonie ausreicht, wobei besagtes Wachstum der Mikrokolonie als Wachstumsphase bezeichnet wird;
Übertragung des porösen Elements und aller darüber befindlichen Medien aus dem Behälter auf ein sekundäres Medium zwecks Evaluierung, Detektion und Identifizierung der Mikroorganismen, wobei besagte Evaluierung, Detektion und Identifizierung der Mikroorganismen als Indikatorphase bezeichnet wird; und
Untersuchung der Mikrokolonien auf Wachstum, Detektion, Identifizierung oder Zählung von Mikroorganismen, wobei besagtes Verfahren zur Züchtung, Detektion und Identifizierung oder Zählung von Mikroorganismen bis zu sechs Stunden dauert, wobei die Wachstumsphase und die Indikatorphase für optimale Ergebnisse voneinander getrennt sind und wobei die Wachstumsphase schnelles Zellwachstum ohne schädliche Indikatoren darin, die das Wachstum verlangsamen, ermöglicht und die Indikatorphase dedizierte Einfärbung und Identifizierung ermöglicht, wodurch kleinere Mikrokolonien effektiv schnell detektiert werden können.

2. Verfahren nach Anspruch 1, wobei das poröse Element eine permeable Membran ist, die aus der Gruppe ausgewählt wird, die aus Polymeren, wie beispielsweise regenerierter Zellulose, Zellophan oder Cuprophan und Dialysemembranen besteht.

3. Verfahren nach Anspruch 1, wobei die Untersuchung der Mikrokolonien zur Identifizierung von Mikroorganismen mit Hilfe von monoklonalen Antikörpern dient.

4. Verfahren nach Anspruch 1, wobei der Untersuchungsschritt manuell oder mit einem automatischen Bilddetektions- und -erkennungssystem durchgeführt wird.

5. Verfahren nach Anspruch 1, ferner umfassend den Schritt des Detektierens der Gesamtzahl lebensfähiger Mikrokolonien (TVO).

6. Verfahren nach Anspruch 1, wobei das sekundäre Medium eine Indikatorsubstanz enthält, um die Identifizierung von Mikroorganismen auf dem porösen Element zu unterstützen.

7. Verfahren nach Anspruch 1, wobei das poröse Element entweder nicht filtrierbare oder filtrierbare Proben einfängt.

8. Verwendung einer Vorrichtung in einem Verfahren nach einem beliebigen der Ansprüche 1 bis 7, umfassend:
einen Nährmediumbehälter zur Bereitstellung von Nährstoffen zur Aufrechterhaltung des Wachstums von Mikroorganismen;
ein poröses, das Nährmedium berührendes Element, wobei das poröse Element das Nährmedium durchlässt, um das Zellwachstum aufrechtzuerhalten, wobei das poröse Element zur späteren Verarbeitung mit Indikatoren aus dem Behälter übertragbar ist und das poröse Element pelluzid, wasserdurchlässig und nährstoffdurchlässig ist.

9. Verwendung einer Vorrichtung nach Anspruch 8, wobei das poröse Element eine permeable Membran ist, die für Mikroorganismen undurchlässig ist, aber autoklavierbar, hydrophil, nicht fluoreszierend, farblos und gegen abgesonderte zelluläre Enzyme beständig ist.

10. Verwendung einer Vorrichtung nach Anspruch 8, wobei das poröse Element über dem Medium in dem Behälter mit einer oder ohne eine obere(n) Schicht Zusatzmedium angeordnet ist.

11. Verwendung einer Vorrichtung nach Anspruch 8, wobei das poröse Element eine permeable Membran mit regelmäßigen oder nicht regelmäßigen Poren im Bereich von 10⁶-10⁷ Da ist.

12. Verwendung einer Vorrichtung nach Anspruch 8, wobei das Medium in einem festen, halbfesten oder flüssigen Zustand vorliegt und wobei das poröse Element Nährstoffe aus dem Medium an Mikroorganismen, die sich über oder auf dem porösen Element befinden, überträgt.

13. Verwendung einer Vorrichtung nach Anspruch 8, ferner umfassend:
eine Mehrzahl von porösen Elementen, die auf oder in dem Medium in einer parallelen Anordnung zur parallelen Prüfung angeordnet sind.

14. Test-Kit-System zur Züchtung, Detektion und Identifizierung oder Zählung von Mikroorganismen, wobei das Test-Kit Folgendes umfasst:
eine in einem beliebigen der Ansprüche 8 bis 13 definierte Vorrichtung;
ein sekundäres Medium, das als Träger zur Freisetzung von Indikatormolekülen in Farbkolonien fungiert; und
eine Bionanokanal-Detektionsplatte mit einer Vielzahl von zylindrischen und parallelen Mikrokanälen, die von einem Ende oder beiden Enden her offen sind, wobei jeder Mikrokanal einem Durchmesser von 1 µm bis 30 µm und eine Länge von 100 µm to 1.000 µm aufweist.

15. Test-Kit-System nach Anspruch 4, wobei das sekundäre Medium aus der Gruppe ausgewählt wird, die aus Agarose, Carrageen, Gelatine und einem Gel mit Befähigung zur Übertragung besteht, und wobei die Konzentration von Gel von 0,1 % bis 10 % in einem Lösungsmittel aus Wasser, Puffer, Natriumchloridlösung oder flüssigem Nährmedium reicht.

## Revendications

1. Procédé consistant à cultiver, détecter et identifier ou énumérer des micro-organismes, comprenant les étapes consistant à :
apporter un récipient d'un milieu nutritif avec un élément poreux disposé sur ou sous la surface du milieu, le milieu renfermant des nutriments destinés à maintenir la croissance de micro-organismes, et l'élément poreux comportant des pores de l'ordre de 1000 à 107 Daltons ;
verser un échantillon liquide dans le récipient dans une zone au-dessus de l'élément poreux ;
piéger les micro-organismes dans l'élément poreux ou sur le milieu au-dessus de l'élément poreux ;
faire incuber suffisamment longtemps le récipient pour cultiver une microcolonie, ladite culture de la microcolonie désignant l'étape de culture ;
transférer l'élément poreux et le milieu situé au-dessus de lui du récipient à un milieu secondaire à des fins d'évaluation, de détection et d'identification des micro-organismes, ladite évaluation, ladite détection et ladite identification des micro-organismes désignant l'étape d'indication ; et
examiner les microcolonies afin de cultiver, détecter et identifier ou énumérer les micro-organismes, ledit procédé de culture, de détection et d'identification ou énumération des micro-organismes durant jusqu'à six heures, l'étape de culture et l'étape d'indication étant séparées pour des résultats optimaux, l'étape de culture permettant une culture cellulaire rapide sans indicateurs préjudiciables qui retardent la culture, et l'étape d'indication permettant une coloration et une identification spécifiques qui permettent une détection rapide et efficace de microcolonies de petite taille.

2. Procédé selon la revendication 1, dans lequel l'élément poreux est une membrane perméable choisie dans le groupe constitué de polymères, tels que la cellulose régénérée, la cellophane ou la cuprophane, et de membranes de dialyse.

3. Procédé selon la revendication 1, dans lequel l'examen des microcolonies consiste à identifier des micro-organismes au moyen d'anticorps monoclonaux.

4. Procédé selon la revendication 1, dans lequel l'étape d'examen est effectuée ou au moyen d'un système automatisé de détection et de reconnaissance d'image.

5. Procédé selon la revendication 1, comprenant en outre l'étape consistant à détecter le nombre total de microcolonies viables (TVO).

6. Procédé selon la revendication 1, dans lequel le milieu secondaire contient une substance indicatrice pour faciliter l'identification des micro-organismes sur l'élément poreux.

7. Procédé selon la revendication 1, dans lequel l'élément poreux piège des échantillons filtrables ou non filtrables.

8. Utilisation d'un dispositif dans un procédé selon l'une quelconque des revendications 1 à 7, comprenant :
un récipient d'un milieu nutritif pour apporter des nutriments afin de maintenir la culture de micro-organismes ;
un élément poreux en contact avec le milieu nutritif, l'élément poreux permettant au milieu nutritif de le traverser afin de maintenir la culture cellulaire, l'élément poreux étant transférable du récipient en vue d'un traitement ultérieur au moyen d'indicateurs, et l'élément poreux étant pellucide, perméable à l'eau et perméable aux substances nutritives.

9. Utilisation d'un dispositif selon la revendication 8, dans lequel l'élément poreux est une membrane perméable qui est imperméable aux micro-organismes, mais qui est autoclavable, hydrophile, non fluorescent, sans couleur et résistant aux enzymes cellulaires sécrétées.

10. Utilisation d'un dispositif selon la revendication 8, dans lequel l'élément poreux est disposé au-dessus du milieu dans le récipient avec ou sans couche supérieure d'un milieu supplémentaire.

11. Utilisation d'un dispositif selon la revendication 8, dans lequel l'élément poreux est une membrane perméable présentant des pores réguliers ou non réguliers de l'ordre de 106 à 107 Da.

12. Utilisation d'un dispositif selon la revendication 8, dans lequel le milieu est à l'état solide, semi-solide ou liquide, et dans lequel l'élément poreux communique les nutriments du milieu aux micro-organismes situés au-dessus ou sur l'élément poreux.

13. Utilisation d'un périphérique selon la revendication 8, comprenant en outre :
une pluralité d'éléments poreux disposés sur ou dans le milieu dans une disposition parallèle en vue d'un test parallèle.

14. Système de kit de test pour la culture, la détection et l'identification ou l'énumération de micro-organismes, le kit de test comprenant :
un dispositif selon l'une quelconque des revendications 8 à 13 ;
un milieu secondaire servant de support pour libérer les molécules indicatrices afin de colorer les colonies ; et
une plaque de détection BioNanoChannel comportant de multiples microcanaux cylindriques et parallèles ouverts à une ou aux deux extrémités, chaque microcanal ayant un diamètre de 1 µm à 30 µm et une longueur de 100 µm à 1000 µm.

15. Système de kit de test selon la revendication 4, dans lequel le milieu secondaire est choisi dans le groupe constitué d'agarose, de carraghénane, de gélatine et d'un gel capable de communiquer, la concentration du gel allant de 0,1 % à 10 % dans un solvant d'eau, un tampon, une solution de chlorure de sodium ou un milieu nutritif liquide.
